(19)

Europäisches Patentamt

European Patent Office

Office européen des brevets

(11) **EP 3 750 408 A1**

(12) **EUROPEAN PATENT APPLICATION**

(43) Date of publication:
   **16.12.2020 Bulletin 2020/51**

(51) Int Cl.:
   **A23K 10/10** (2016.01)      **A23L 11/00** (2016.01)
   **A23L 11/30** (2016.01)

(21) Application number: **19179984.0**

(22) Date of filing: **13.06.2019**

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   Designated Validation States:
   **KH MA MD TN**

(71) Applicant: **Bofood Aktiebolag
   374 31 Karlshamn (SE)**

(72) Inventors:
   • **Berggren Söderlund, Gunilla Anna Maria
     222 74 LUND (SE)**

   • **Andersson, Kenneth Lars-Olof
     247 35 SÖDRA SANDBY (SE)**
   • **Funeteg, Bo Åke Ingolf
     293 91 OLOFSTRÖM (SE)**
   • **Lindvall, Kent Inge Stephan
     216 13 LIMHAMN (SE)**
   • **Sjöberg, Klas Göran
     217 48 MALMÖ (SE)**
   • **Söderlund, Bengt Patrik
     222 74 LUND (SE)**

(74) Representative: **Brann AB
   P.O. Box 3690
   Drottninggatan 27
   103 59 Stockholm (SE)**

(54) **METHODS OF PROCESSING A FAVA BEAN COMPOSITION**

(57)    A method of processing a fava bean composition is disclosed, the method comprising the steps of: i. providing a fava bean composition comprising fava beans, and ii. subjecting the fava bean composition to a betaglucosidase and/or at least one lactobacillus plantarum bacteria under dosage, time and temperature conditions selected so as to be sufficient to reduce the content of at least one of the compounds vicine and convicine, and preferably also at least one of the compounds divicine and isouramil, by at least 90 wt% as compared to the content of said compounds in the fava bean composition provided in step i. A fava bean composition obtained by the method as well as methods of producing food compositions using the fava bean composition and food compositions thus produced are also disclosed.

Fig. 1A

EP 3 750 408 A1

**Description**

TECHNICAL FIELD

The technology proposed herein relates to the field of methods of processing fava bean compositions to produce fava bean compositions that are safe to ingest for people having glucose-6-phosphate dehydrogenase deficiency (G6PDD). More particularly the technology proposed herein relates to the field of methods for processing fava bean compositions to decrease or remove the content of substances which, when ingested as part of the fava bean composition, causes or may cause favism, i.e. acute haemolysis.

BACKGROUND

Glucose-6-phosphate dehydrogenase deficiency (G6PDD) (favism) is an inborn error of metabolism that predisposes to red blood cell breakdown. Following a specific trigger, symptoms such as yellowish skin, dark urine, shortness of breath, and feeling tired may develop. Complications can include inter alia anemia. This disorder is an X-linked recessive disorder resulting in a defective glucose-6-phosphate dehydrogenase enzyme and the red blood cell breakdown may be triggered by infections, certain medication, or in particular ingestion of fava beans.

It is estimated that around 400 million people have G6PDD worldwide, and the condition is particularly common in parts of Africa, Asia, the Middle East, and the Mediterranean.

The degree of deficiency varies between different individuals, spanning from those who are asymptotic, who do not suffer the red blood cell breakdown, to those who are symptomatic, i.e. who suffer the red cell breakdown and the associated hemolytic condition.

As suggested by the name *favism*, the fava bean (*Vicia faba*), also called broad bean or faba bean, is one type of food that can trigger the breakdown of red blood cells in individuals with G6PDD. The fava bean is a species of flowering plants in the bean and pea family. This bean is cultivated widely around the world for human consumption and can be consumed raw or cooked. Fava beans are rich in carbohydrates (58 %) and protein (26 %) and further contain considerable amounts of vitamins and minerals.

It has been suggested that the compounds vicine, divicine, convicine and isouramil (these compounds being capable of creating oxidants in the red blood cells) found in the fava bean are responsible for the hemolytic reaction in an individual having G6PDD when ingesting fava beans.

Thus, while the nutritious properties and global spread would motivate further cultivation and use of fava beans and fava bean compositions for human consumption, the toxicity and risk it poses to individuals with G6PDD, many of which may not know that they have the condition until exposed to fava beans, prevents a barrier to such further use.

Accordingly it is an object of the technology proposed herein to provide a method by which a fava bean composition, i.e. a composition comprising fava beans, is processed so as to at least lessen the risk that it triggers a hemolytic reaction when consumed by an individual suffering from G6PDD.

More particularly it is an object of the technology proposed herein to provide a method by which the content of at least one of the compounds vicine and convicine, and preferably also at least one of the compounds divicine and isouramil, is reduced at least by 90 wt% as compared to the corresponding content in the non-processed fava bean.

Further objects of the technology proposed herein relate to the provision of food products comprising a fava bean composition, as well as methods of producing the food products.

SUMMARY

At least one of the above mentioned objects are, according to the first aspect of the technology proposed herein achieved by

A method of processing a fava bean composition, comprising the steps of:

    i. providing a fava bean composition comprising fava beans, and
    ii. subjecting the fava bean composition to a betaglucosidase and/or at least one lactobacillus plantarum bacteria under dosage, time and temperature conditions selected so as to be sufficient to reduce the content of at least one of the compounds vicine and convicine, and preferably also at least one of the compounds divicine and isouramil, by at least 90 wt% as compared to the content of said compounds in the fava bean composition provided in step i.

Thus the technology proposed herein is based on the realization that an enzymation using betaglucosidase and/or a fermentation using a lactobacillus plantarum bacteria can sufficiently lower the content of at least one of the compounds vicine and convicine, and preferably also the at least one of the compounds divicine and isouramil, in a fava bean composition, so that the risk that an individual having G6PDD reacts to the fava bean composition is at least decreased, or that the extent or severity of the individual's reaction to consuming the fava bean composition is at least decreased.

Additionally the technology proposed herein is based on the recognition that, whereas fava beans generally contain a high content of vicine and convicine and a low content of the divicine and isouramil, typical or conventional food processing technologies, such as heat treatments for reasons of microbial control or other biological or enzymatic process steps, may in fact cause the degradation of the vicine and convicine. This degradation results in the formation of divicine and isouramil. Accordingly a fava bean composition that has been processed using typical or conventional food processing technologies may come to contain a higher amount of the more potent compounds divicine and isouramil than the fava bean itself.

The term processing is to be understood as encompassing the terms treating.

The term fava bean composition is intended to encompass compositions comprising fava beans. A fava bean composition may thus for example comprise or consist of fava beans which preferably are crushed or milled. Preferably a fava bean composition comprises crushed or milled fava beans in a suspension or liquid such as water. The fava beans may be crushed or milled or disintegrated to particles less than 5 mm in diameter, such as less than 3 mm, less than 1 mm or less than 0.5 mm.

The fava bean composition may further comprise other foodstuffs, such as for example cereals (for example oats). Accordingly it must be understood that other foodstuffs may be included in, or added to, the fava bean composition before, during, and after the steps of the method.

The fava bean composition may be prepared or produced as a step in the method.

The term fava bean encompasses the terms Vicia faba, broad bean and faba bean.

The fava bean composition may be subjected to the betaglucosidase and/or the lactobacillus plantarum bacteria for example in a mixing tank or enzymation/fermentation reactor. The mixing tank or enzymation/fermentation reactor may for example be provided with a stirrer and/or devices for heating and/or cooling the contents of the mixing tank or reactor.

Accordingly subjecting encompasses bringing into contact with and/or mixing with.

The dosage of betaglucosidase, i.e. the amount of betaglucosidase that is added to the fava bean composition, is dependent on the desired time for the enzymation, the temperature during the enzymation, the amount of fava bean composition (and its dry matter content) to be processed, and the activity of the enzyme. Generally this dosage should be at least 0.05 wt% based on the amount of fava bean composition that is to be processed. The dosage is preferably from 0.1 to 1 wt% when processing a fava bean composition having a dry matter content of 9 wt% using a betaglucosidase with an activity of 1500 AZO BBG U/g or alternatively at least 6200IU/g. The dosage may be proportionally decreased or increased when a betaglucosidase having a higher, respectively a lower, activity is used. The dosage may further be proportionally decreased or increased when the dry matter content of the fava bean composition is lower or higher. However, instead of decreasing or increasing the dosage the time or temperature conditions may be proportionally decreased or increased.

The activity of the enzyme may be at least 1500 AZO BBG U/g or alternatively at least 6200IU/g. If the activity of the enzyme is increased the dosage may be correspondingly decreased, and vice versa.

Betaglucosidase as used herein encompasses both solutions of betaglucosidase, such as a 15-20 Wt% of betaglucosidase in solution, as well as isolated (100 wt%) betaglucosidase. Where a solution with a higher concentration of betaglucosidase, or isolated betaglucosidase is used, and the activity correspondingly increases, the dosage is preferably correspondingly decreased. Accordingly the dosage, based on the weight of isolated betaglucosidase used, may be from may be from 0.01 wt%, such as 0.02 to 0.2 wt%.

Betaglucosidase encompasses any enzyme having the function of a betaglucosidase, i.e. that of hydrolysing glycosidic bonds to terminal non-reducing residues in glucosides and oligosaccharides, with release of glucose.

The betaglucosidase may be an enzyme complex hydrolysing beta glucans and non-starch polysaccharides like arabinoxylans. The betaglucosidase may preferably be a cellulase produced by fermentation with Trichoderma reesei.

For the enzymation the temperature condition is generally from 40-65°C, preferably 65 °C, and the time conditions are typically 2 to 12 hours, preferably 2 hours.

The dosage of the at least one lactobacillus plantarum bacteria, i.e. the amount of the lactobacillus plantarum bacteria that is added to the fava bean composition, is dependent on the desired time for the fermentation, the temperature during the fermentation, and the amount of fava bean composition (and its dry matter content). Typically this dosage may be from 0.01 wt% to 0.1 wt%, such as for example 0.02 wt%, based on the amount of fava bean composition that is to be processed when the dry matter content of the fava bean composition is 9 wt%. The dosage may be proportionally decreased or increased when the dry matter content of the fava bean composition is lower or higher. However, instead of decreasing or increasing the dosage the time or temperature conditions may be proportionally decreased or increased.

The temperature conditions for the fermentation are typically 36-40 °C, preferably 37-39 °C, such as preferably 38°C. The time conditions for the fermentation is typically 6-16 hours, preferably 10-16 hours, more preferably 11-13 hours, such as preferably 12 hours.

The pH during the fermentation may preferably be from 3.5 to 5 such as from 3.8 to 4.6.

The dosage, time and temperature conditions are to be selected so that the sufficient reduction of least one of the compounds vicine and convicine is obtained. Generally a higher temperature increases the reaction rate of the reaction. With an increased reaction rate the time needed for obtaining the sufficient reduction decreases, whereas a lower temperature requires longer time to obtain the sufficient reduction. Likewise and increased dosage decreases the time needed to obtain the sufficient reduction. The time and temperature conditions refers to the time that the betaglucosidase and/or the at least one bacillus plantarum bacteria is in contact with the fava bean composition and capable of enzymatically and/or by fermentation affecting the fava bean composition, as well as the temperature of the reaction mixture of the fava bean composition and the betaglucosidase during the reaction, i.e. during step (ii).

The dosage, time and temperature conditions may be selected so as to be sufficient to reduce the content of both of the compounds vicine and convicine, and preferably also both of the compounds divicine and isouramil, by at least 90 wt% as compared to the content of said compounds in the fava bean composition provided in step i.

Preferably the dosage, time and temperature conditions are selected so as to be sufficient to reduce the sum content of the compounds vicine and convicine, and preferably also the sum content of the compounds divicine and isouramil, by at least 90 wt% as compared to the content of said compounds in the fava bean composition provided in step i.

More preferably the dosage, time and temperature conditions are selected so as to be sufficient to reduce the sum content of the compounds vicine, convicine, divicine, and isouramil, by at least 90 wt% as compared to the content of said compounds in the fava bean composition provided in step i.

The reduction is at least 90 wt%. More preferably the reduction is at least 94 wt%, 95 wt% or more, such as 96, 97, 98, 99, 99.5, 99.9, 99.95, 99.99 wt%.

Preferably the fava bean composition provided in step i has been heat treated for reducing the microbial content or for sterilizing the fava bean composition, or the method further comprises a step of:

iii. heat treating the fava bean composition for reducing the microbial content of the fava bean composition or for sterilizing the fava bean composition.

The reduction of the microbial content of the fava bean composition, or the sterilization the fava bean composition, may be advantageous for using the fava bean composition as a foodstuff. In this case the method preferably comprises subjecting the fava bean composition to the betaglucosidase as the betaglucosidase is effective in breaking down divicine and isouramil which may be formed by degradation of vicine and convicine during heat treatment, see inter alia sample 3 in table showing that subjecting the fava bean composition to the betaglucosidase results in a low content of divicine and isouramil.

This advantage is obtained both when the fava bean composition is provided in step i already heat treated, and when the fava bean composition is heat treated as the step iii of the method before or after step ii.

The heat treatment may comprise pasteurizing, UHT treatment, and/or autoclaving.

Further the method may also comprise a step of:

iv. reducing the microbial content of the fava bean composition, or sterilizing the fava bean composition, using irradiation sterilisation or gas sterilisation.

Thus, other methods for reducing the microbial content or for sterilizing the fava bean composition may also be utilized together with, or instead of, heat treatment.

The fava bean composition provided in step i may comprise crushed, milled and/or homogenized fava beans in an aqueous liquid, preferably water.

As fava beans contain about 11% water, a fava bean composition can be obtained by merely crushing, milling and/or homogenizing fava bean, thus obtaining a composition comprising about 11wt% water. The composition can generally have a minimum content of water of at least 5 wt% by for example removing water from the fava bean composition. It is generally preferred however to increase the water content of the fava bean composition by adding further water to the fava beans. The weight ratio of fava beans to added water (weight of fava beans : weight of water) may for example be from 9.5:1 to 1:9.5, such as from 9:1 to 1:9. Preferably the weight ratio may be from 3:1 to 1:3. Further or alternatively the dry matter content of the fava bean composition may be from 2% to 88%, such as from 5 to 50%, such as from 5 to 20%, preferably from 5 to 15 %, such as 7 to 12 %, such as 8 -10%.

Preferably the dosage, time and temperature conditions are selected so as to be sufficient to decrease the sum content of vicine and convicine in the fava bean composition to less than 6 wt%, preferably less than 2 wt%, more preferably less than 1 wt% of the sum content of said compounds in the fava bean composition provided in step i, or alternatively selected so as to be sufficient to decrease the sum content of vicine and convicine in the fava bean composition to less than 65 mg/kg, preferably less than 22 mg/kg, more preferably less than 12 mg/kg of the fava bean composition. The sum contents of 6 wt%, preferably less than 2 wt%, more preferably less than 1 wt%, correspond to reductions of at least 94 wt%, preferably at least 98wt%, and more preferably at least 99 wt%.

Additionally or alternatively the dosage, time and temperature conditions may be selected so as to be sufficient to decrease the sum content of divicine and isouramil in the fava bean composition to less than 150 mg/kg of the fava bean composition, preferably less than 50 mg/kg of the fava bean composition, more preferably less than 10 mg/kg of the fava bean composition, such as less than 1 mg/kg of the fava bean composition, such as less than 0.1 mg/kg or less than 0.01 mg/kg of the fava bean composition.

Preferably step ii comprises subjecting the fava bean composition to a betaglucosidase, wherein the dosage is at least 0.1 wt% based on the amount of the fava bean composition, and wherein the time and temperature conditions are at least 2 hours and at least 60 °C. Preferably the dosage is from 0.1 wt% to 1 wt%, such as from 0.15 wt% to 1 wt% or from 0.25 wt% to 1 wt% such as from 0.25 wt% to 0.5 wt%, based on the amount, i.e. weight, of the fava bean composition. The activity of the betaglucosidase is preferably 1500 AZO BBG U/g or alternatively at least 6200IU/g. As shown by the samples in table 1 use of betaglucosidase, alone or in in combination with the lactobacillus plantarum bacteria, ensures low contents of at least one of the compounds vicine, convicine, divicine and isouramil.

Alternatively step ii comprises subjecting the fava bean composition to at least one lactobacillus plantarum bacteria, wherein the dosage is at least 0.01 wt%, such as 0.01 to 0.1 wt% based on the amount of the fava bean composition, and wherein the time and temperature conditions are at least 10 hours, such as at least 12 hours, and at least 35 °C.

A fermentation step in which the fava bean composition is subjected to at least one lactobacillus plantarum bacteria also decreases the content of at least one of the compounds vicine, convicine, divicine and isouramil.

Lactobacillus plantarum bacteria encompasses bacteria selected from the strain lactobacillus plantarum.

Preferably step ii comprises subjecting the fava bean composition to both the betaglucosidase and the at least one lactobacillus plantarum bacteria, wherein the fava bean composition is subjected to the betaglucosidase under first dosage, time and temperature conditions, and subjected to the lactobacillus plantarum bacteria under second dosage, time and temperature conditions.

The respective first and second dosage, time and temperature conditions may be as given for the betaglucosidase and the at least one lactobacillus plantarum bacteria above or initially.

The fava bean composition may be subjected to the betaglucosidase and the lactobacillus plantarum bacteria sequentially.

Preferably however step ii comprises subjecting the fava bean composition simultaneously to the betaglucosidase and the at least one lactobacillus plantarum bacteria, wherein the first and second time and temperature conditions are the same. As seen in the table, sample 6, this results in a very low content of all of vicine, convicine, divicine and isouramil.

In this case the first and second time and temperature conditions are the same and may be at least 10 hours, such as 10-16 hours, and at least 35 °C, such as 35-40 °C, preferably 37-39 °C or 38 °C.

The at least one bacteria from the strain lactobacillus plantarum may be *L. plantarum* 299 or *L. plantarum* 299v.

These bacterial strains are examples of strains suitable for the fermentation.

Preferably the first and second dosage, time and temperature conditions are selected so as to be sufficient for reducing the total sum content of vicine, convicine, divicine and isouramil, calculated using the formula:

total sum content = sum content of vicine and convicine + 30*sum content of divicine and isouramil,

to less than 1500 mg/kg of the fava bean composition, preferably less than 340 mg/kg of the fava bean composition.

This is for example obtained in samples 5 and 6.

Alternatively the second dosage, time and temperature conditions should be selected so that the content of at least one of vicine and convicine, and preferably also at least one of divicine and isouramil, is reduced more when step ii of the method includes subjecting the fava bean composition to both the betaglucosidase and the at least one lactobacillus bacteria than when step ii includes only one subjecting the fava bean composition to only one of the betaglucosidase and the at least one lactobacillus bacteria.

The method may further comprise the step of:

vi. collecting a solid fraction of the fava bean composition, such as by decanting, whereby the remainder of the fava bean composition defines a liquid fraction of the fava bean composition.

The solid fraction may be used for different purposes, such as (part of) a meat substitute. The solid fraction may be used inter alia for dairy substitutes, beverages, in soups, etc. Step vi may be performed before or after steps ii and v.

A second aspect of the technology proposed herein further concerns a fava bean composition obtained by the method according to the first aspect of the technology proposed herein.

The fava bean composition is characterized in that it has a reduced content of at least one of vicine and convicine in accordance with the reduction obtained in the method used to process the fava bean composition as described above. Preferably the fava bean composition has a sum content of

A third aspect of the technology proposed herein further concerns a method of manufacturing a food product or a feed product comprising using the fava bean composition according to the second aspect of the technology proposed herein.

A wide variety of food products and feed products may be produced from the fava bean composition processed according to the method. The fava bean composition may be a major part of such products, or alternatively it may be a minor part of the products.

The method according to third aspect of the technology proposed herein may further comprise any of:

vii. adding the fava bean composition, or the solid fraction or the liquid fraction thereof, to an oat-derived or oat-comprising composition,

viii. using the liquid fraction of the fava bean composition to produce an ice-cream, a whippable cream, or a beverage, and

ix. using the solid fraction of the fava bean composition to produce a meat substitute.

This provides suitable food products comprising fava bean compositions. The solid fraction may further be used to produce a feed product.

A fourth aspect of the technology proposed herein further concerns a food product or feed product obtained by the method according to the third aspect of the technology proposed herein.

The food product or feed product may for example be in the form of an ice-cream, whippable cream or beverage. The food product or feed product is characterized in that it has a reduced content of at least one of vicine and convicine in accordance with the reduction obtained in the method used to process the fava bean composition as described above.

BRIEF DESCRIPTION OF THE FIGURES AND DETAILED DESCRIPTION

A more complete understanding of the abovementioned and other features and advantages of the technology proposed herein will be apparent from the following detailed description of preferred embodiments in conjunction with the appended drawings, wherein:

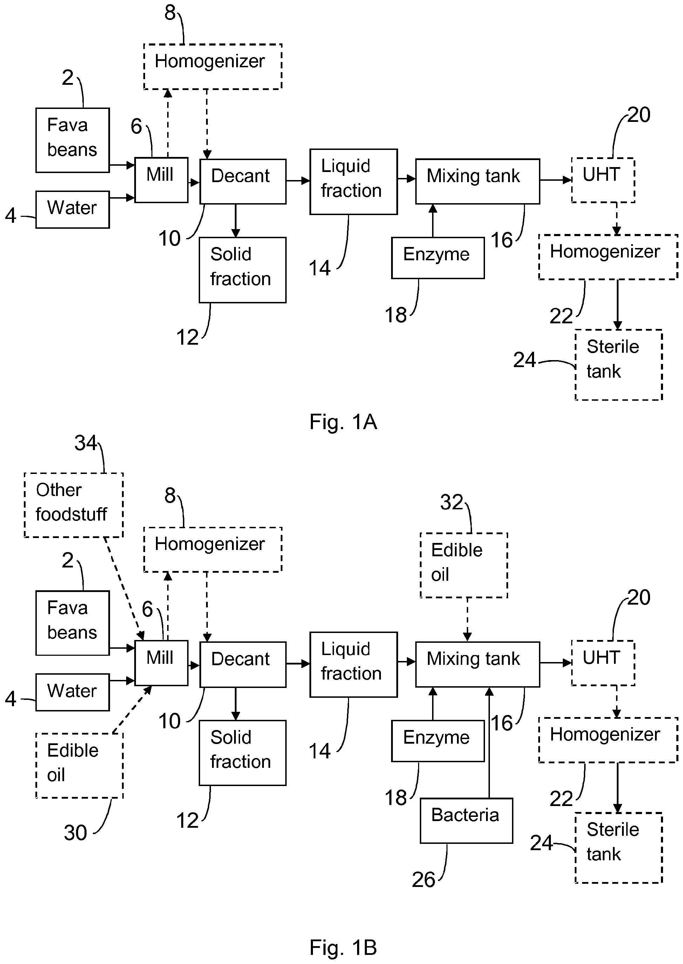

Fig. 1A     shows inter alia a first embodiment of the method of processing a fava bean composition according to the first aspect of the technology proposed herein,

Fig. 1B     shows inter alia a second embodiment of the method of processing a fava bean composition according to the first aspect of the technology proposed herein

In the below description of the figures the same reference numerals are used to designate the same features throughout the figures. Further, where present a 'added to a reference numeral indicates that the feature is a variant of the feature designated with the corresponding reference numeral not carrying the '-sign.

Fig. 1A shows inter alia a first embodiment of the method of processing a fava bean composition according to the first aspect of the technology proposed herein. As shown in the figure fava beans 2 and water 4 are admitted to a mill 6 in which the fava beans are milled and finely divided together with the water. The fava beans are disintegrated to particles less than 5 mm in diameter. The milling in the mill 6 may take place at any suitable temperature. Typically the temperature is at least 20 °C. The temperature may be higher, such as at least 70°C or 90°C, for example 95°C temperature. The milling continues until of the fava beans 2 have been reduced in size to the size provided by the mill 6, i.e. less than 5 mm diameter. The mixture of milled fava beans, i.e. the fava bean material, and water is hereinafter referred to as a fava bean composition. The fava bean composition here has a dry matter content of about 9 wt%. The fava bean composition may optionally, after the mill 6, be homogenized in a homogenizer 8 at elevated pressure and temperature, for example at more than 70 °C or more than 80 °C and at more than 100 bar or any other pressure suitable to further disintegrate the fava bean material in the fava bean composition and to increase the degree of extraction of the nutrients (carbohydrates, proteins, minerals, vitamins, etc.) from the fava bean material.

The fava bean composition is then decanted in decanter 10 for separating out a solid fraction 12 of the fava bean composition. This solid fraction 12 may be used as described further below.

The remainder of the fava bean composition, the liquid fraction 14, is transferred into a mixing tank 16. In order to reduce the content of the compounds vicine, divicine, convicine and isouramil in the liquid fraction 14 a betaglucosidase enzyme 18 is added to the mixing tank 16. The addition of the enzyme 18 is typically 0.1 to 1 wt% of the fava bean composition and the enzyme is allowed to process the liquid fraction 14 for about at least 1 hour, such as for example 2-4 hours and at a temperature, such as from 40-70 °C, for example from 55 to 65 °C, at which the enzyme is active. The enzyme may, as here, be provided as a solution containing 20 wt% enzyme, whereby the dosage of 0.1 to 1 wt% refers to the enzyme solution and corresponds to 0.02 to 0.2 wt% of isolated enzyme being added.

The so treated liquid fraction comprises a reduced content of at least one of the compounds vicine and convicine.

Optionally, to reduce the microbial content, the liquid fraction may for example be heat treated, for example as illustrated here using an UHT device 20 wherein it is heat treated at temperatures above 100 °C for sufficient time.

If then enzyme is not inactivated in the UHT device 20, then the enzyme may be deactivated in the mixing tank 16, after the enzymation process has concluded by cooling the mixing tank 16 to a temperature below room temperature, or alternatively be heating the mixing tank 16 to a temperature sufficient to cause heat denaturing of the enzyme.

Optionally the liquid fraction 14 may be homogenized in a second homogenizer 22 before being stored, for example in a sterile tank 24, or used further.

The liquid fraction 14 of the fava bean composition so obtained may be used as a substitute to cream and whipping cream, as a base for soups etc.

The solid fraction 12 may be used as a meat substitute.

Both the solid fraction 12 and the liquid fraction 14 contain reduced contents of the compounds vicine, divicine, convicine and isouramil as compared to the fava beans 2 in the beginning of the process.

Although in fig 1A only the liquid fraction 14 enters the mixing tank 16 to be enzymated using the enzyme 18, it is also possible to position the decanter 10 after the mixing tank 16 such that the enzymation using the enzyme 18 is performed on the fava bean composition prior to the separation of the fava bean composition into the solid fraction 12 and the liquid fraction 14.

Further, whereas fig 1A (and 1B below) show and describe the use of a number of devices and steps, including the mill 6, the homogenizer 8, decanter 10, UHT device 20, homogenizer 22, sterile tank 24, which are advantageous, these devices and steps are not essential for achieving the objects of the technology described herein.

Fig. 1B shows inter alia a second embodiment of the method of processing a fava bean composition according to the first aspect of the technology proposed herein. This embodiment differs in that, in addition to the enzyme 18 and the following enzymation in the mixing tank 16, fermentation is also performed in the mixing tank 16 following the addition of a lactobacillus plantarum strain bacteria 26 (*L. plantarum* 299). Specifically Lactobacillus plantarum DSM 9843 can be used.

In a first alternative embodiment the bacteria 26 is added prior to the addition of the enzyme 18. The liquid fraction 14 is thus allowed to ferment for 12-24 hours (preferably 12 hours) at a temperature of 35-40 °C (such as 38 °C). Thereafter the enzyme 18 is added and the enzymation takes place at 30-70 °C (such as 60 °C) for a time of 1-12 hours (such as 2 hours).

In a second alternative embodiment the bacteria 26 is added at the same time as the enzyme 18. The liquid fraction 14 in the mixing tank 16 is then simultaneously fermented and enzymated for at least 6 hours at a temperature of 35-40 °C (such as 38 °C).

In certain cases it may be desired to further adjust or increase the nutritious properties of the fava bean composition. Edible oil, such as rape seed oil, may in particular be added to the mixing tank upon conclusion of the enzymation and fermentation.

This option is illustrated in Fig. 1B by the addition of edible oil 30 either in the beginning of the process, or the addition of edible oil 32 to the mixing tank 16. Edible oil may additionally be added at other steps of the method.

Other foodstuffs may also be added to the fava bean composition. One example is oats and other cereals. Foodstuffs may in particular be added prior to the addition of the enzyme 18 and or the bacteria 26, for example as indicated by 34 in Fig. 1B.

EXAMPLE

Table 1 disclose the results obtained on reducing the content of vicine, divicine, convicine and isouramil in a fava bean composition (liquid fraction) dependent on the treatment method used.

The content of the compounds was determined as follows.

Vicine and convicine were determined as based on the method described by Gutierrez et al: CAPs markers to assist selection for low vicine and convicine contents in faba bean (Vicia faba L.). Theor. Appl. Genet. 2006. The samples were extracted with water in a hot water bath for 3.5 hours, after which concentrated HCl was added to the sample extracts. Samples were analyzed by an Agilent 1100 series high performance liquid chromatography device with a diode array detector (HPLC-DAD). A C18 column was used with a gradient of phosphate buffer and methanol. The breakdown products divicine and isouramil were identified by their UV-spectra and quantitated using a calibration curve of vicine and correcting the result by molar mass ratio.

Table 1

| Sample no. | Method/sample | Contents | Comment |
|---|---|---|---|
| 1 | Freshly harvested fava bean | In total about 10740 mg/kg of convicine and vicine | Reference value for a non-processed fava bean. Divicine and isouramil are degradation products/metabolites of convicine and vicine, and therefore not present in any significant amount in the fava bean. |
| 2 | Fava bean composition as formed according to fig. 1A but without the enzymation step. (Temperature of fava bean composition during processing about 90 °C) | 360 mg/kg of convicine<br><br>791 mg/kg of vicine (in total about 1079 mg/kg of convicine and vicine)<br><br>Less than 2 mg/kg of divicine and isouramil | The fava bean composition comprises a lower content of convicine and vicine compared to the freshly harvested fava bean. This is due to the dilution effected by the addition of the water 4. In this case the ratio of fava<br><br>bean:water was about 1:9. Although the content of convicine and vicine has been reduced compared to the freshly harvested fava bean, it however needs to be further reduced. |
| 3 | The liquid fraction obtained as shown in fig 1A, i.e. using only enzymation.<br><br>(The temperature of fava bean composition in the steps up to the enzymation was about 90 °C. Enzymation using 0.25 wt% dosage of betaglucosidase (activity of 1500 AZO BBG U/g or 6200IU/g) for 2 hours at 60 °C) | 5 mg/kg of convicine 68 mg/kg of vicine divicine could not detected (less than 2 mg/kg) isouramil could not be detected (less than 2 mg/kg) | This process provides a significant reduction of convicine and vicine. A total reduction of about 93 % has been obtained (corresponding to ((5 mg + 68 mg) / 1079 mg)). No divicine or isouramil could be detected in the fava bean composition. |
| 4 | The liquid fraction obtained as shown in fig 1A with the difference that fermentation alone was used instead of enzymation.<br><br>(The temperature of fava bean composition in the steps up to the fermentation was about 90 °C. Fermentation using 0.02 wt% dosage for 12 hours at 38 °C) | 12 mg/kg of convicine 4 mg/kg of vicine In total 134 mg/kg of divicine and isouramil | This process reduces convicine and vicine more than the process using enzymation; however the content of divicine and isouramil has increased. A total reduction of about 86% has been obtained (corresponding to ((12 mg + 4 mg + 134 mg)/ 1079 mg). |

(continued)

| Sample no. | Method/sample | Contents | Comment |
|---|---|---|---|
| 5 | The liquid fraction obtained as shown in fig 1B: first alternative in which fermentation and enzymation are performed sequentially. (Temperature of fava bean composition in the steps up to fermentation was about 90 °C. fermentation: 0.02 wt% for 12 hours at 38 °C), enzymation: 0.25 wt% for 2 hours at 60 °C). | 2 mg/kg of convicine, 21 mg/kg of vicine, In total 46 mg/kg of divicine and isouramil | The sequential fermentation and enzymation reduces the content of convicine and vicine further in comparison to enzymation only, however the sum content of divicine and isouramil is higher. A total reduction of about 93% has been obtained (corresponding to ((2 mg + 21 mg + 46 mg)/ 1079 mg) |
| 6 | The liquid fraction obtained as shown in fig 1 B: second alternative in which enzymation and fermentation performed concurrently. (Temperature of fava bean composition in the steps up to fermentation was about 90 °C. Combined fermentation (0.02 wt%) and enzymation: 0.25 wt% for 12 hours at 38 °C) | 10 mg/kg of convicine, 2 mg/kg of vicine, In total no mg/kg of divicine and isouramil could | Concurrent enzymation and fermentation yields the lowest contents of convicine and divicine, together with a low sum content of divicine and isouramil. A total reduction of about 99% has been obtained (corresponding to ((10 mg + 2 mg + 0 mg)/ 1079 mg) |

Divicine and isouramil are about 30 times more potent in causing symptoms in individuals with G6PDD. Accordingly it becomes clear that at least an enzymation step using betaglucosidase is preferably used for processing the fava bean composition. The addition of the fermentation step, as shown in Fig. 1B, further decreases the content of convicine and vicine, especially when, as in sample 6, the enzymation is performed during the time that the fermentation is performed.

**Claims**

1. A method of processing a fava bean composition, comprising the steps of:

   i. providing a fava bean composition comprising fava beans, and
   ii. subjecting the fava bean composition to a betaglucosidase and/or at least one lactobacillus plantarum bacteria under dosage, time and temperature conditions selected so as to be sufficient to reduce the content of at least one of the compounds vicine and convicine, and preferably also at least one of the compounds divicine and isouramil, by at least 90 wt% as compared to the content of said compounds in the fava bean composition provided in step i.

2. The method according to claim 1, wherein the fava bean composition provided in step i has been heat treated for reducing the microbial content or for sterilizing the fava bean composition, or wherein the method further comprises a step of:

   iv. heat treating the fava bean composition for reducing the microbial content of the fava bean composition or for sterilizing the fava bean composition.

3. The method according to any preceding claim, wherein the fava bean composition provided in step i comprises crushed, milled and/or homogenized fava beans in an aqueous liquid.

4. The method according to any preceding claim, wherein the dosage, time and temperature conditions are selected so as to be sufficient to decrease the sum content of vicine and convicine in the fava bean composition to less than 6 wt%, preferably less than 2 wt%, more preferably less than 1 wt% of the sum content of said compounds in the fava bean composition provided in step i, or alternatively selected so as to be sufficient to decrease the sum content of vicine and convicine in the fava bean composition to less than 65 mg/kg, preferably less than 22 mg/kg, more preferably less than 12 mg/kg of the fava bean composition.

5. The method according to any preceding claim, wherein the dosage, time and temperature conditions are selected so as to be sufficient to decrease the sum content of divicine and isouramil in the fava bean composition to less than 150 mg/kg of the fava bean composition, preferably less than 50 mg/kg of the fava bean composition, more preferably less than 10 mg/kg of the fava bean composition, such as less than 1 mg/kg of the fava bean composition, such as less than 0.1 mg/kg or less than 0.01 mg/kg of the fava bean composition..

6. The method according to any of the preceding claims, wherein step ii comprises subjecting the fava bean composition to the betaglucosidase, wherein the dosage is at least 0.1 wt% based on the amount of the fava bean composition, and wherein the time and temperature conditions are at least 2 hours and at least 60 °C.

7. The method according to any of the claims 1-5, wherein step ii comprises subjecting the fava bean composition to the at least one lactobacillus plantarum bacteria, wherein the dosage is at least 0.01 wt%, such as 0.01 to 0.1 wt% based on the amount of the fava bean composition, and wherein the time and temperature conditions are at least 10 hours, such as at least 12 hours, and at least 35 °C

8. The method according to any preceding claim, wherein step ii comprises subjecting the fava bean composition to both the betaglucosidase and the at least one lactobacillus plantarum bacteria, wherein the fava bean composition is subjected to the betaglucosidase under first dosage, time and temperature conditions, and subjected to the lactobacillus plantarum bacteria under second dosage, time and temperature conditions.

9. The method according to claim 8, wherein step ii comprises subjecting the fava bean composition simultaneously to the betaglucosidase and the at least one lactobacillus plantarum bacteria, and wherein the first and second time and temperature conditions are the same.

10. The method according to any of the preceding claims, wherein the at least one lactobacillus plantarum bacteria is *L. plantarum* 299 or *L. plantarum* 299v.

11. The method according to any of the claims 8-10, wherein the first and second dosage, time and temperature conditions are selected so as to be sufficient for reducing the total sum content of vicine, convicine, divicine and isouramil, calculated using the formula:

total sum content = sum content of vicine and convicine + 30*sum content of divicine and isouramil

to less than 1500 mg/kg of the fava bean composition, preferably less than 340 mg/kg of the fava bean composition.

12. The method according to any of the preceding claims, further comprising the step of:

vii. collecting a solid fraction of the fava bean composition, such as by decanting, whereby the remainder of the fava bean composition defines a liquid fraction of the fava bean composition.

13. A fava bean composition obtained by the method according to any of the preceding claims.

14. A method of manufacturing a food product or a feed product comprising using the fava bean composition according to claim 13.

15. A food product or feed product obtained by the method according to claim 14.

Fig. 1A

Fig. 1B

Europäisches
Patentamt

European
Patent Office

Office européen
des brevets

## EUROPEAN SEARCH REPORT

Application Number

EP 19 17 9984

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/158959 A1 (TEKNOLOGIAN TUTKIMUSKESKUS VTT OY [FI]) 22 October 2015 (2015-10-22) * the whole document * | 1,3, 7-10, 12-15 | INV. A23K10/10 A23L11/00 A23L11/30 |
| X | CODA ROSSANA ET AL: "Effect of air classification and fermentation byLactobacillus plantarumVTT E-133328 on faba bean (Vicia fabaL.) flour nutritional properties", INTERNATIONAL JOURNAL OF FOOD MICROBIOLOGY, vol. 193, 12 October 2014 (2014-10-12), pages 34-42, XP029112345, ELSEVIER BV, NL ISSN: 0168-1605, DOI: 10.1016/J.IJFOODMICRO.2014.10.012 * the whole document * | 1,3, 8-10, 12-15 | |
| X | ARBID M S S; MARQUARDT R R: "HYDROLYSIS OF THE TOXIC CONSTITUENTS VICINE AND CONVICINE IN FABA BEAN VICIA-FABA FOOD PREPARATIONS FOLLOWING TREATMENT WITH BETA GLUCOSIDASE", JOURNAL OF THE SCIENCE OF FOOD AND AGRICULTURE, vol. 36, 1985, pages 839-846, XP002796319, * the whole document * | 1-6, 12-15 | |

**TECHNICAL FIELDS SEARCHED (IPC)**

A23K
A23L

-/--

The present search report has been drawn up for all claims

| Place of search | Date of completion of the search | Examiner |
|---|---|---|
| The Hague | 9 December 2019 | Piret-Viprey, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

| | Europäisches Patentamt European Patent Office Office européen des brevets | **EUROPEAN SEARCH REPORT** | **Application Number** EP 19 17 9984 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A.M. MCKAY: "Hydrolysis of vicine and convicine from fababeans by microbial beta-glucosidase enzymes", JOURNAL OF APPLIED BACTERIOLOGY, vol. 72, no. 6, 1 June 1992 (1992-06-01), pages 475-478, XP055231617, GB ISSN: 0021-8847, DOI: 10.1111/j.1365-2672.1992.tb01861.x * the whole document * | 1-4, 7-10, 12-15 | |
| X | RIZZELLO CARLO GIUSEPPE ET AL: "Degradation of vicine, convicine and their aglycones during fermentation of faba bean flour", SCIENTIFIC REPORTS, vol. 6, 31 August 2016 (2016-08-31), pages 1-11, XP002796320, ISSN: 2045-2322, DOI: 10.1038/srep32452 * the whole document * | 1,3-5, 7-15 | |
| X | PULKKINEN MARJO ET AL: "Determination and stability of divicine and isouramil produced by enzymatic hydrolysis of vicine and convicine of faba bean", FOOD CHEMISTRY, vol. 212, 13 May 2016 (2016-05-13), pages 10-19, XP029627570, ELSEVIER LTD, NL ISSN: 0308-8146, DOI: 10.1016/J.FOODCHEM.2016.05.077 * the whole document * | 1,3-5, 13-15 | TECHNICAL FIELDS SEARCHED (IPC) |
| X A | US 2018/255803 A1 (SPINELLI MICHAEL A [US] ET AL) 13 September 2018 (2018-09-13) * the whole document * | 13-15 12 | |
| | -/-- | | |

The present search report has been drawn up for all claims

| Place of search | Date of completion of the search | Examiner |
|---|---|---|
| The Hague | 9 December 2019 | Piret-Viprey, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 19 17 9984

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | W.J. PITZ, F.W. SOSULSKI: "Determination of Vicine and Convincine in Fababean Cultivars by Gas-Liquid Chromatography", CANADIAN INSTITUTE OF FOOD SCIENCE AND TECHNOLOGY JOURNAL, vol. 12, no. 2, January 1979 (1979-01), pages 93-97, XP002796321, * table 3 * | 13 | |
| X | K. ELKOWICZ ET AL: "Antinutritive Factors in Eleven Legumes and Their Air-classified Protein and Starch Fractions", JOURNAL OF FOOD SCIENCE, vol. 47, no. 4, 1 July 1982 (1982-07-01), pages 1301-1304, XP055231619, US ISSN: 0022-1147, DOI: 10.1111/j.1365-2621.1982.tb07673.x * table 4 * * page 1303, right-hand column, paragraph 4-5 * | 13 | |
| A | JAKUBCZYK ANNA ET AL: "Peptides obtained from fermented faba bean seeds (Vicia faba) as potential inhibitors of an enzyme involved in the pathogenesis of metabolic syndrome", LWT- FOOD SCIENCE AND TECHNOLOGY, vol. 105, 10 February 2019 (2019-02-10), pages 306-313, XP085625990, ISSN: 0023-6438, DOI: 10.1016/J.LWT.2019.02.009 * page 307, left-hand column, paragraph 3 * * page 307, left-hand column, last paragraph * * table 2 * | 10 | |

TECHNICAL FIELDS
SEARCHED (IPC)

The present search report has been drawn up for all claims

| Place of search | Date of completion of the search | Examiner |
|---|---|---|
| The Hague | 9 December 2019 | Piret-Viprey, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 9984

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2019

| Patent document cited in search report | Publication date | Patent family member(s) | Publication date |
|---|---|---|---|
| WO 2015158959 A1 | 22-10-2015 | NONE | |
| US 2018255803 A1 | 13-09-2018 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUTIERREZ et al.** CAPs markers to assist selection for low vicine and convicine contents in faba bean (Vicia faba L.). *Theor. Appl. Genet.*, 2006